# EUROPÄISCHE PATENTSCHRIFT

(11) **EP 0 755 678 B1**
(45) Veröffentlichungstag und Bekanntmachung des Hinweises auf die Patenterteilung: **02.01.2003**
(21) Anmeldenummer: 96109709.4
(22) Anmeldetag: 17.06.1996
(51) Int. Cl.: A61K 31/165, A61K 31/245, A61P 27/16

(54) **Methode und Zusammensetzung einer topischen Therapie von Innenohr und Labyrinth-Symptomen**
Method and composition for a topical therapy of symptoms of the inner ear and the labyrinth
Méthode et composition pour une thérapie topique de symptomes de l'oreille interne et du labyrinthe

(30) Priorität: 06.07.1995 DE 19524691
(43) Veröffentlichungstag der Anmeldung: 29.01.1997
(73) Patentinhaber: EpiCept Corporation, Englewood Cliffs, N.J. 07632 (US)
(72) Erfinder: Liedtke, Rainer K., Dr. c/c Pharmed Techn. GmbH, 82027 Grünwald (DE)
(74) Vertreter: Leson, Thomas Johannes Alois, Dipl.-Ing.

(56) Entgegenhaltungen:
- EP-A- 0 331 392
- EP-A- 0 488 137
- WO-A-92/15289
- "Rote Liste 1994" 1994 , BUNDESVERBAND DER PHARMAZEUTISCHEN INDUSTRIE , EDITIO CANTOR, AULENDORF/WÜRTT., BRD XP002018927 *Otologika, 68001-68017*
- ACTA OTOLARYNGOL, Bd. 112, Nr. 5, 1992, Seiten 800-809, XP000609357 E. LAURIKAINEN ET AL.: "Experimental basis for lidocaine therapy in cochlear disorders"
- CHEMICAL ABSTRACTS, vol. 122, no. 23, 5.Juni 1995 Columbus, Ohio, US; abstract no. 282062h, K. OGAWA ET AL.: "Effects of lidocaine on phosphoinositide hydrolysis in the cochlear sensory epithelia of the rat. Implications to be the mechanism of tinnitus suppression with lidocaine." Seite 109; XP002018928 & OTOL. JPN, Bd. 4, Nr. 5, 1994, Seiten 651-658,
- EAR AND HEARING, Bd. 17, Nr. 1, Februar 1996, Seiten 49-54, XP000609352 E. LAURIAKAINEN ET AL.: "Effects of intratympanically delivered lidocaine on the auditory system in humans"
- AVIATION SPACE & ENVIRONMENTAL MEDICINE, Bd. 56, Nr. 8, August 1985, Seiten 777-782, XP0006009346 I. PYYKKÖ ET AL.: "The effects of TTS-scopolamine, dimenhydramine, lidocaine, and tocainide on motion sickness, vertigo, and nystagmus"

## Beschreibung

Die Erfindung betrifft die Verwendung eines Lokalanästhetikums zur Herstellung einer Zusammensetzung für die topischen Therapie pathologischer Symptomatiken des Innenohrs und Labyrinths, insbesondere von Ohrgeräuschen, Schwindel, Gleichgewichtstörungen und Nausea.

Es ist bekannt, daß persistierende Ohrengeräusche, sowie Schwindelerscheinungen mit Gleichgewichtstörungen und Nausea, meist auf Störung oder Erkrankung der Organe des Innenohrs oder auch der Hörnerven beruhen. Ohrgeräusche können in niederfrequenten wie hochfrequenten Qualitäten auftreten, niederfreguent dabei insbesondere bei Erkankungen von Gehörgang und Mittelohr, hochfrequente insbesondere bei Störungen des Labyrinth. Diese anhaltenden Symptome belasten die Betroffenen erheblichst. Einen in dieser Hinsicht charakteristischen Symptomenkomplex, mit Ohrensausen, Drehschwindel, Gleichgewichtsstörungen, evtl. verbunden mit Nystagmus, Schwerhörigkeit und Erbrechen, stellt beispielweise der Morbus Menière dar. Die hierbei attackenweise auftretende Symptomatik kann auf vasomotorischen Störungen von Labyrinth-Gefäßen oder vorübergehenden Störungen von Sekretion und Zusammensetzung des Labyrinth Liquors beruhen. Die Betroffenen werden im Laufe dieser Krankheit unter dem Einfluß permanenter Ohrengeräusche wie auch Schwerhörigkeit oft reizbar und ängstlich, mit teils erheblichen psychosomatischen Problematiken.

Die bisherigen Therapien stellen noch keine ausreichend wirksamen Behandlungsformen dar. Sofern eine Grundkrankheit nicht eruierbar sind nur symptomatische Maßnahmen wie Reizdeprivation, Ruhe und pharmakologische Sedation möglich. Als pharmakologisches Prinzip wird hierzu häufig das sedativ wirkende orale Dimenhydrinat eingesetzt. Auch das Parasympatholytikum Scopolamin wird eingesetzt. Dessen Einsatz beinhaltet aber primär die, pathophysiologisch zusammhängende, Erscheinung bewegungsinduzierter Übelkeit, der Kinetose, die bei Exposition in von außen bewegten Objekten entsteht. Sie ist auch bekannt als Reise- oder Seekrankheit, die mit vegetativen Erscheinungen, vor allem aber mit Nausea verbunden ist. Hier wird zur systemischen Therapie auch eine transdermale Route für Scopolamin eingesetzt (Chien, Y.W., Novel Drug Delivery Systems; Drugs and the Pharmaceutical Sciences; Vol.14 , 1982, Marcel Dekker, New York), womit ein niedrigerer und gleichmäßigerer Blutspiegel im Körper erzielt wird als mit intramuskulärer Scopolamin Verabreichung. Trotzdem zeigt auch dies die typisch unerwünschten Nebenwirkungen von Scopolamin, insbesondere Sehstörungen, starke Mundtrockenheit, Änderungen im Konzentrationsverhalten und Schläfrigkeit. Letzere unerwünschte Wirkung ist u.a. auch der bei dem gleichfalls bei Kinetosen eingesetzten, aber weniger wirksamen, oralen Dimenhydrinat der Fall. Insgesamt ist somit die Anwendung von Scopolamin als Therapieprinzip nur sehr restriktiv handhabbar.

Als besser wirksames pharmakologisches Prinzip ist eine geeignete Anwendung von niedrig dosierten Lokalalänästhetika zu betrachten. Doch könnte hier eine systemische Anwendung nur mit Injektion erfolgen, was von daher schon weitgehend und auf Grund der Gefahr systemischer Überdosierung mit ernsten kardialen Nebenwirkungen, entfällt. Eine direkte Anwendung von Lokalananästhetika am labyrinthären Apparat des Innenohrs selbst ist technisch praktisch ausgeschlossen, zudem bestünde Gefahr ototoxischer Effekte.

Amid- und Ester-Lokalanästhetika, z.B Lidocain vom Amid-Typ, zeigen als pharmakologischen Wirkungsmechanismus eine Hemmung des schnellen Natrium Ionen-Influx in Nervenfasern. Sie blockieren hierüber die Impulsleitung der Nervenbahn, was prinzipiell alle regionalen Nervenfasern betrifft. Die dünneren sensorischen Fasern sind aber, infolge ihrer Morphologie, empfindlicher als motorische Fasern, woraus sich Wirkungseffekte differenzieren lassen. Bekannt ist auch, daß sich bei intravenösen Injektionen höherer Dosen Lidocain (Gejrot, T., Atl. Lokalanästhesie, S.151-152, Thieme, Stuttgart 1970) sowie auch einer Blockade des Ganglion Stellatum mit Procain, positive Einflüsse auf Symptome von Morbus Meniere zeigten, allerdings zeitlich limitiert.

Eine nichtinvasive Therapie pathologischer Symptomatiken des Innenohrs oder Labyrinths mit einem topischen Trägersystem wurde bisher weder vorgenommen noch beschrieben.

Die EP-A-0 331 392 betrifft ein Trägersystem, in welches ein Lokalanästhetikum wie Lidocain eingebracht ist und bei dem eine anästhetische und antimikrobielle Wirkung für die Haut selbst erreicht werden soll.

Die EP-A-0 488 137 beschreibt ein transdermales Trägersystem, welches in der Haftschicht ein Lokalanästhetikum in einem sehr hohen Anteil von 40 - 65 Gew.-% enthält, wobei ein spezielles Verhältnis zwischen ungelöstem und gelöstem Lokalanästhetikum in der Haftschicht eingehalten sein muss, wobei es vornehmlich darum geht, eine möglichst rasche Betäubungswirkung zu erreichen. Eine wirksamen Behandlung spezieller pathologischer Zustände des Innenohrs und Labyrinths wird nicht erwähnt.

Die WO-A-92/15 289 offenbart Zusammensetzungen zur topischen Applikation von pharmazeutisch wirksamen Mitteln über formbeständige Träger. Als pharmazeutisch wirksame Mittel werden Lokalanästhetika, aber auch eine Vielzahl weiterer pharmakologischer Prinzipien in Betracht gezogen.

Die Veröffentlichung von W.A. Laurikainen et al. in ACTA OTOLARYNGOL, Bd. 112, S. 800-809 (1992) beschreibt eine experimentelle Basis für eine Therapie durch Lidocain, d.h. einem Lokalanästhetikum, in Bezug auf Cochlea-Beschwerden wie die Meniere's Krankheit und Tinnitus. Dabei wird die Wirkungen des Lidocains in Bezug auf den Blutfluss und die Elektrophysiologie des Innenohrs (Cochlea) in einem experimentellen Tiersystem studiert. Die Applikationsart ist eine intratympanischen Verabreichung durch die RW-Route, was einer Injektion in Innenbereiche des Ohrs entspricht.

Die Veröffentlichung CHEMICAL ABSTRACTS, Vol. 122, Nr. 23, Abstract Nr. 282062h vom 5. Juni 1995 (entsprechend OTOL. JPN., Bd. 4, Nr. 5, S. 651-658 (1994) beschreibt ein Studium am Tiermodell, bei dem die Wirkungen von Lidocain auf die Phosphoinositid-Hydrolyse in den sensorischen Epithelien des Cochleas der Ratte beschrieben werden. Es werden Implikationen für die Erleichterung von Tinnitus mittels Lidocain diskutiert, ohne ein Konzept zur wirksamen Behandlung pathologischer Symptome des Innenohrs und des Labyrinths darzustellen.

Der Erfindung liegt die Aufgabe zugrunde, die Therapie pathologischer Symptomatiken des Innenohrs oder Labyrinths mit einem nichtinvasiven topischen Anwendungssystem zu verbessern.

Diese Aufgabe wird dadurch gelöst, daß ein Lokalanästhetikums zur Herstellung einer Zusammensetzung für die Therapie pathologischer Symptome des Innenohrs und Labyrinths verwendet wird, wobei die Lokalanästhetikumenthaltende Zusammensetzung in ein peri-aurikulär topisches Trägersystem mit therapeutischer Dosis eines Lokalanästhetikums eingebracht wird, wobei das peri-aurikuläre topische Trägersystem in der Lage ist, auf der intakten Haut angebracht zu werden und das enthaltene Lokalanästhetikum auf die unter dem Träger liegende peri-aurikuläre Hautregion freizusetzen.

Um die Therapie zu erweitern, werden in einer weiteren Ausbildung der Erfindung, als neue therapeutische Anwendungebiete für ein peri-aurikulär topischen Trägersystem mit Lokalanästhetika insbesondere Ohrgeräusche, Schwindel, Gleichgewichtstörungen, Kinetosen und Nausea einbezogen.

Um Wirksamkeit und Verträglichkeit der peri-aurikulär topischen Therapie zu verbessern, werden in einer weiteren Ausbildung der Erfindung Lokalanästhetika vom Amid- oder Ester-Typ verwendet, insbesondere Lidocain, Tetracain, Bupivacain, Prilocain, Mepivacain sowie Procain und Benzocain, wobei diese Stoffe in Konzentrationsbereichen von 0,5%-40% vorliegen.

Um Wirksamkeit und Verträglichkeit der Therapie zu verbessern werden, in weiteren Ausbildung der Erfindung, in dem eingesetzten per-aurikulär topischen Trägersytem 2 Lokalanästhetika mit unterschiedlicher Pharmakokinetik kombiniert, wobei diese Einzelstoffe in solchen Konzentrationen vorliegen, daß die Gesamtkonzentration beider Wirkstoffe nicht mehr als 40% beträgt.

Um die Therapie insgesamt sicherer und besser handhabbar zu machen, liegt, in einer weiteren Ausbildung der Erfindung, das peri-aurikulär topische Trägersystem in solchen Formen vor, die den speziellen Gegebenheiten von Morphologie und Anatomie des peri-aurikulären Applikationsfeldes entsprechen, wobei die äußere Form des topischen Trägersystems rund, oval, eckig oder halbmondförmig, mit konkaven oder konvexen Aussenformen ist, oder das Trägersystem auch, mit oder ohne zusätzliche Hilfsmittel, vom Anwender in entsprechende Formen segmentiert werden kann.

Eine Therapie von pathologischen Symptomatiken des Innenohrs und Labyrinths mittels eines peri-aurikulär topisch applizierten Trägersystem mit Lokalanästhetika wurde bisher weder vorgenommen noch beschrieben worden. Ein mit der Erfindung erzielter Vorteil ergibt sich daher, daß mit diesem neuen pharmakologisch-technischen Prinzip erstmals auch eine nichtinvasive topische Behandlungsmöglichkeit von belastenden Symptomatiken des Innenohr- und Labyrinth-Organs gegeben ist, für die auch bisher noch keine adäquate Therapie vorliegt.

Die peri-aurikulär topische Therapie mit Lokalanästhetika in einem Trägersystem ermöglicht eine lokal gezielte und dauerhafte thera peutische Beeinflussung von terminal und funktionell vernetzten Nervenbahnen.

Da der peri-aurikuläre Bereich über gute cutane Resorptionskapazität verfügt, kann zudem mit nur geringen topischen Dosierungen vorgegangen werden. Systemische Gefährdung wird vermieden, da Lokalanästhetika, z.B. Lidocain, bei der retardierten cutanen Aufnahme weit überwiegend metabolisiert werden, so daß auch keine systemischen Wirkspiegel auftreten.
Die Therapie ist über geringe Dosen steuerbar und längeranhaltend und kann nach Bedarf durch Abnahme des Trägers unterbrochen werden.
Insgesamt hat die peri-aurikuläre topische Therapie auch weniger systemische Nebeneffekte als systemische Therapien wie Diphenhydramin oder Scopolamin, da nicht vorherige Wirkssoff-Distribution über den ganzen Körper erfolgt und die Lokalanästehika zudem auch über eine gute lokale Gewebeverträglichkeit verfügen.

Als Beispiel für eine technisch geeignete Ausgestaltung der erfindungsgemäßen Verwendung in einem peri-aurikulären topischen Trägersystems mit Lokalanästhetika wird auf die Beschreibungen der technischen Trägersyteme in US-No. 4,765,986 und EP 0205974 verwiesen, jedoch ohne es damit auf diese beschriebenen Techniken beschränken zu wollen.

Zur Therapie von pathologischen Symtomen des Innenohres und Labyrinths werden peri-aurikuläre topische Trägersysteme mit Lokalanästhetika eingesetzt.

Die Anwendung dieser Therapieform bezieht dabei insbesondere und als neue Indikationen eine nichinvaise topische Behandlung von Ohrgeräuschen, Schwindel, Gleichgewichtsstörungen und Nausea ein.

Die peri-aurikulär topische Therapie gemäß der erfindungsgemäßen Verwendung in einem Trägersystem mit Lokalanästhetika stellt eine neue nebenwirkungsarme und effektive Behandlungsmöglichkeit bei Störungen des Innenohrs und Labyrinths dar.

## Patentansprüche

1. Verwendung eines Lokalanästhetikums zur Herstellung einer Zusammensetzung für die Therapie pathologischer Symptome des Innenohrs und Labyrinths, wobei die Lokalanästhetikum-enthaltende Zusammensetzung in ein peri-aurikuläres topisches Trägersystem mit einer therapeutischen Dosis des Lokalanästhetikums eingebracht wird, wobei das peri-aurikuläre topische Trägersystem in der Lage ist, auf der intakten Haut angebracht zu werden und das enthaltene Lokalanästhetikum auf die unter dem Träger liegende peri-aurikuläre Hautregion freizusetzen.

2. Verwendung nach Anspruch 1, **dadurch gekennzeichnet, dass** als neue medizinische Anwendungsgebiete für ein peri-aurikuläres topisches Trägersystem mit Lokalanästhetika insbesondere Ohrgeräusche, Schwindel, Gleichgewichtsstörungen, Kinetosen und Nausea einbezogen sind.

3. Verwendung nach vorhergehendem Anspruch 1 oder 2, **dadurch gekennzeichnet, dass** in dem peri-aurikulären topischen Trägersystem Lokalanästhetika vom Amid- oder Ester-Typ eingesetzt werden, insbesondere Lidocain, Prilocain, Bupivacain, Mepivacain sowie Procain und Benzocain, wobei diese Stoffe in Konzentrationsbereichen von 0,5% - 40% vorliegen.

4. Verwendung nach einem der vorhergehenden Ansprüche, **dadurch gekennzeichnet, dass** in dem eingesetzten peri-aurikulären topischen Trägersystem 2 Lokalanästhetika mit unterschiedlicher Pharmakokinetik kombiniert sind, wobei diese Einzelstoffe in solchen Konzentrationen vorliegen, dass die Gesamtkonzentration beider Wirkstoffe nicht mehr als 40% beträgt.

5. Verwendung nach einem der vorhergehenden Ansprüche, **dadurch gekennzeichnet, dass** das peri-aurikuläre topische Trägersystem in einer solchen Form vorliegt, die den speziellen Gegebenheiten von Morphologie und Anatomie des peri-aurikulären Applikationsfeldes entspricht, wobei die äußere Form des topischen Trägersystems rund, oval, eckig oder halbmondförmig, mit konkaven oder konvexen Außenformen ist, oder das Trägersystem, mit oder ohne zusätzliche Hilfsmittel, vom Anwender in entsprechende Formen segmentiert werden kann.

## Revendications

1. Utilisation d'un anesthésique local pour préparer une composition pour la thérapie de symptômes pathologiques de l'oreille interne et du labyrinthe, la composition contenant l'anesthésique local étant introduite dans un système de support topique péri-auriculaire avec une dose thérapeutique de l'anesthésique local, le système de support topique péri-auriculaire étant en mesure d'être appliqué sur la peau intacte et de libérer l'anesthésique contenu sur la région de la peau péri-auriculaire située sous le support.

2. Utilisation selon la revendication 1, **caractérisée en ce que** les nouveaux domaines médicaux d'application d'un système de support topique péri-auricuiaire avec anesthésique local comprennent en particulier les acufènes, les vertiges, les troubles de l'équilibre, les kinétoses et les nausées.

3. Utilisation selon la revendication 1 ou 2 précédentes, **caractérisée en ce qu'**on utilise dans le système de support topique péri-auriculaire des anesthésiques locaux de type amides ou esters, en particulier lidocaïne, prilocaïne, bupivacaïne, mépivacaïne ainsi que procaïne et benzocaïne, ces substances se trouvant dans des concentrations comprises entre 0,5 % et 40 %.

4. Utilisation selon l'une des revendications précédentes, **caractérisée en ce que** dans le système de support topique péri-auriculaire sont combinés deux anesthésiques locaux à pharmacodynamique différente, chacune de ces substances se trouvant dans des concentrations telles que la concentration totale des deux substances actives ne représente pas plus de 40 %.

5. Utilisation selon l'une des revendications précédentes, **caractérisée en ce que** le système de support topique péri-auriculaire se présente sous une forme qui correspond aux données particulières de la morphologie et de l'anatomie du champ d'application péri-auriculaire, la forme extérieure du système de support topique étant circulaire, ovale, angulaire ou en forme de demi-lune, avec des formes extérieures concaves ou convexes, ou le système de support, avec ou sans moyens auxiliaires supplémentaires, peut être segmenté par l'utilisateur dans des formes appropriées.

## Claims

1. Use of a local anaesthetic for preparation of a composition for therapy of pathological symptoms of the inner ear and labyrinth, wherein the composition containing the local anaesthetic is introduced into a peri-auricular topical carrier system with a therapeutic dose of a local anaesthetic, the peri-auricular topical carrier system being capable of being applied to the intact skin and of releasing the local anaesthetic contained to the peri-auricular skin region lying under the carrier.

2. Use according to Claim 1, **characterized in that** in particular tinnitus, dizziness, imbalance, kinetoses and nausea are included among new medical fields of application for a peri-auricular topical carrier system with local anaesthetics.

3. Use according to preceding Claims 1 or 2, **characterized in that** local anaesthetics of the amide or ester type, in particular lidocaine, prilocaine, bupivacaine and mepivacaine, and also procaine and benzocaine, are used in the peri-auricular topical carrier system, these substances being present in concentrations ranging from 0.5% to 40%.

4. Use according to any one of the preceding claims, **characterized in that** 2 local anaesthetics with different pharmacokinetics are combined in the peri-auricular topical carrier system used, these individual substances being present in such concentrations that the total concentration of both active ingredients is not more than 40%.

5. Use according to any one of the preceding claims, **characterized in that** the peri-auricular topical carrier system is present in a form that corresponds to the special morphological and anatomical features of the peri-auricular application-field, the outer form of the topical carrier system being round, oval, square or half-moon shaped, with concave or convex outer forms, or the carrier system being capable of being segmented into suitable shapes by the user, with or without additional aids.
